# EUROPEAN PATENT APPLICATION

(11) **EP 4 566 990 A1**
(43) Date of publication of application: **11.06.2025**
(21) Application number: 24171221.5
(22) Date of filing: 19.04.2024
(51) Int. Cl.: C01B 33/193, A61K 8/25

(54) **AMORPHOUS SILICA PARTICLE, PREPARATION METHOD AND USE THEREOF**

(30) Priority: 07.12.2023 CN 202311673331
(71) Applicant: Jinsanjiang (Zhaoqing) Silicon Material Co., LTD., Zhaoqing, Guangdong 526200 (CN)
(72) Inventor: CHEN, Ying, Zhaoqing, Guangdong, 526200 (CN); WANG, Xianwei, Zhaoqing, Guangdong, 526200 (CN); HUANG, Dan, Zhaoqing, Guangdong, 526200 (CN); ZHENG, Songling, Zhaoqing, Guangdong, 526200 (CN); LIU, Jing, Zhaoqing, Guangdong, 526200 (CN); MI, Shuang, Zhaoqing, Guangdong, 526200 (CN); CHEN, Guanglong, Zhaoqing, Guangdong, 526200 (CN); WEI, Yanying, Zhaoqing, Guangdong, 526200 (CN); ZHENG, Peiwen, Zhaoqing, Guangdong, 526200 (CN)
(74) Representative: Porta & Consulenti Associati S.p.A.

(57) **Abstract**

An amorphous silica particle, a preparation method and use thereof is disclosed. The amorphous silica particle provided by the present disclosure meets 0.85≤ a Cetyl Trimethyl Ammonium Bromide (CTAB) specific surface area/a Brunauer-Emmett-Teller (BET) specific surface area≤1. The amorphous silica particle provided by the present disclosure has both excellent friction and cleaning abilities and high light transmittance, when used in a toothpaste as an abrasive, the amorphous silica particle can obtain the excellent cleaning effect and the high transparency characteristic with a relatively low addition amount, without causing excessive wear of dentin and enamel, and the amorphous silica particle is suitable for preparing a whitening and transparent toothpaste with high friction and cleaning properties.

## Description

### TECHNICAL FIELD

The present disclosure relates to the technical field of inorganic fillers, and in particular to an amorphous silica particle, a preparation method and use thereof.

### BACKGROUND

With the advantages of stable physical and chemical properties, acid and alkali resistance, high temperature resistance, no pollution to the environment, good cleaning performance, good compatibility with other ingredients in toothpaste, high fluorine compatibility and the like, silica is gradually replacing calcium carbonate, calcium hydrogen phosphate, calcium pyrophosphate and the like to become the main abrasive in the toothpaste. As we all know, the abrasive in the toothpaste with a high cleaning effect will inevitably cause a certain degree of abrasion on the tooth surface, and the abrasion rate of the toothpaste on the teeth must be kept low enough to ensure that the tooth surface, especially the dentin, will not generate a permanent damage due to daily cleaning.

In recent years, transparent toothpastes have become more and more popular, but the toothpastes with both high friction and cleaning ability and high transparency are very rare on the market. The reason is that, from the perspective of the structure of silica, the high friction value and the high transparency are contradictory properties, the silica generally used to make the highly transparent toothpaste will inevitably have the disadvantage of insufficient friction and cleaning performance, and the silica with the high cleaning performance often has the problem of low light transmittance. Therefore, how to overcome the contradictory problem of the high friction value and the high transparency of the silica is one of the problems that needs to be solved urgently.

Therefore, it is of great significance to develop a silica particle that has both excellent friction and cleaning ability and high light transmittance.

### SUMMARY

The objective of the present disclosure is to provide an amorphous silica particle, a preparation method and use thereof.

The technical solution used in the present disclosure is as follows:
An amorphous silica particle, which meets 0.85<a Cetyl Trimethyl Ammonium Bromide (CTAB) specific surface area/a Brunauer-Emmett-Teller (BET) specific surface area≤1.

Preferably, the CTAB specific surface area of the amorphous silica particle is ≤60 m²/g.

Preferably, the BET specific surface area of the amorphous silica particle is ≤70 m²/g.

Preferably, an apparent density of the amorphous silica particle is ≥0.1 g/mL.

Preferably, an oil factor of the amorphous silica particle is ≤300 g/100 g.

A preparation method for the amorphous silica particle as described above includes the following steps:
1) adding a certain amount of water to a reaction tank, heating to 50 °C-90 °C and then starting stirring; adding a certain amount of sodium silicate aqueous solution, and then adding the sodium silicate aqueous solution and a sulfuric acid solution simultaneously at a maintained flow rate until a preset amount of sodium silicate aqueous solution is added, controlling a pH value of a reaction solution to 7-8 and a temperature to 50 °C-75 °C during the reaction, and then stopping adding the sulfuric acid solution for aging;
2) adding a certain amount of sodium silicate aqueous solution to the reaction tank, and then adding the sodium silicate aqueous solution and the sulfuric acid solution at a maintained flow rate until a preset amount of sodium silicates aqueous solution is added, controlling a pH value of a reaction solution to 9-10 and a temperature to 75 °C-90 °C during the reaction, and then stopping adding the sulfuric acid solution for aging; and
3) adding the sulfuric acid solution to the reaction tank until a pH value of a reaction solution is 4-5, and then stopping adding the sulfuric acid solution for aging; drying and then crushing an aged product to obtain the amorphous silica particle.

Preferably, the certain amount of water, the certain amount of sodium silicate aqueous solution and the preset amount of sodium silicate aqueous solution in step 1) have a volume ratio of 1:0.4-0.6:5-10.

Preferably, the stirring in step 1) is performed at a rotation speed of 1200 r/min-2400 r/min.

Preferably, the sodium silicate aqueous solution in step 1) has a concentration of 2.7 N-3.5 N.

Preferably, the sulfuric acid solution in step 1) has a concentration of 18 N-36 N.

Preferably, the aging in step 1) lasts for 15 min-60 min.

Preferably, the certain amount of sodium silicate aqueous solution and the preset amount of sodium silicate aqueous solution in step 2) have a volume ratio of 1: 15-25.

Further preferably, the certain amount of sodium silicate aqueous solution and the preset amount of sodium silicate aqueous solution in step 2) have a volume ratio of 1:20-25.

Preferably, the preset amount of sodium silicate aqueous solution in step 1) and the preset amount of sodium silicate aqueous solution in step 2) have a volume ratio of 1:0.5-4.

Further preferably, the preset amount of sodium silicate aqueous solution in step 1) and the preset amount of sodium silicate aqueous solution in step 2) have a volume ratio of 1:0.5-2.

Preferably, the sodium silicate aqueous solution in step 2) has a concentration of 2.7 N-3.5 N.

Preferably, the sulfuric acid solution in step 2) has a concentration of 18 N-36 N.

Preferably, the aging in step 2) lasts for 15 min-60 min.

Preferably, the sulfuric acid solution in step 3) has a concentration of 18 N-36 N.

Preferably, the aging in step 3) lasts for 15 min-60 min.

An oral cleaning composition, comprising the amorphous silica particle as described above.

Preferably, the oral cleaning composition further comprises an orally acceptable carrier.

Preferably, the amorphous silica particle has a weight percentage of 10%-30%.

Preferably, the oral cleaning composition is a toothpaste.

The beneficial effects of the present disclosure are as follows: the amorphous silica particle provided by the present disclosure has both excellent friction and cleaning ability and high light transmittance, when used in a toothpaste as an abrasive, the amorphous silica particle can obtain the excellent cleaning effect and the high transparency characteristic with a relatively low addition amount, without causing excessive wear of dentin and enamel, and the amorphous silica particle is suitable for preparing a whitening and transparent toothpaste with high friction and cleaning properties.

### DETAILED DESCRIPTION

The present disclosure will be further explained and described below in conjunction with specific examples.

### Example 1

An amorphous silica particle, which was prepared by a preparation method as follows:
1) 2 L of water was added to a reaction tank and heated to 55 °C, and then stirring was started at a rotation speed of 1700 r/min; 1 L of sodium silicate aqueous solution with a concentration of 2.7 N was added, then the sodium silicate aqueous solution with a concentration of 2.7 N and a sulfuric acid solution with a concentration of 18 N were added simultaneously until a preset amount of sodium silicate aqueous solution was added, wherein the sodium silicate aqueous solution had a preset amount of 20 L and a flow rate of 0.33 L/min; a pH value of a reaction solution was controlled to 7-8 and a temperature was controlled to 55 °C-60 °C during the reaction, and then the sulfuric acid solution was stopped adding for aging for 60 min;
2) 2 L of sodium silicate aqueous solution with a concentration of 2.7 N was added to the reaction tank, then the sodium silicate aqueous solution with a concentration of 2.7 N and the sulfuric acid solution with a concentration of 18 N were added simultaneously until a preset amount of sodium silicate aqueous solution was added, wherein the sodium silicate aqueous solution had a preset amount of 40 L and a flow rate of 1 L/min; a pH value of a reaction solution was controlled to 9-10 and a temperature was controlled to 85 °C-90 °C during the reaction, and then the sulfuric acid solution was stopped adding for aging for 45 min; and
3) the sulfuric acid solution with a concentration of 18 N was added to the reaction tank until a pH value of a reaction solution was 4.5, then the sulfuric acid solution was stopped adding for aging for 15 min, and then drying and crushing were performed on an aged product to obtain the amorphous silica particle.

### Example 2

An amorphous silica particle, which was prepared by a preparation method as follows:
1) 4 L of water was added to a reaction tank and heated to 60 °C, and then stirring was started at a rotation speed of 1700 r/min; 2 L of sodium silicate aqueous solution with a concentration of 2.9 N was added, then the sodium silicate aqueous solution with a concentration of 2.9 N and a sulfuric acid solution with a concentration of 24 N were added simultaneously until a preset amount of sodium silicate aqueous solution was added, wherein the sodium silicate aqueous solution had a preset amount of 40 L and a flow rate of 0.66 L/min; a pH value of a reaction solution was controlled to 7-8 and a temperature was controlled to 60 °C-65 °C during the reaction, and then the sulfuric acid solution was stopped adding for aging for 60 min;
2) 1.8 L of sodium silicate aqueous solution with a concentration of 2.9 N was added to the reaction tank, then the sodium silicate aqueous solution with a concentration of 2.9 N and the sulfuric acid solution with a concentration of 24 N were added simultaneously until a preset amount of sodium silicate aqueous solution was added, wherein the sodium silicate aqueous solution had a preset amount of 45 L and a flow rate of 1.5 L/min; a pH value of a reaction solution was controlled to 9-10 and a temperature was controlled to 80 °C-85 °C during the reaction, and then the sulfuric acid solution was stopped adding for aging for 45 min; and
3) the sulfuric acid solution with a concentration of 24 N was added to the reaction tank until a pH value of a reaction solution was 4.5, then the sulfuric acid solution was stopped adding for aging for 15 min, and then drying and crushing were performed on an aged product to obtain the amorphous silica particle.

### Example 3

An amorphous silica particle, which was prepared by a preparation method as follows:
1) 6 L of water was added to a reaction tank and heated to 65 °C, and then stirring was started at a rotation speed of 1700 r/min; 3 L of sodium silicate aqueous solution with a concentration of 3.1 N was added, then the sodium silicate aqueous solution with a concentration of 3.1 N and a sulfuric acid solution with a concentration of 28 N were added simultaneously until a preset amount of sodium silicate aqueous solution was added, wherein the sodium silicate aqueous solution had a preset amount of 60 L and a flow rate of 1 L/min; a pH value of a reaction solution was controlled to 7-8 and a temperature was controlled to 65 °C-70 °C during the reaction, and then the sulfuric acid solution was stopped adding for aging for 60 min;
2) 1.7 L of sodium silicate aqueous solution with a concentration of 3.1 N was added to the reaction tank, then the sodium silicate aqueous solution with a concentration of 3.1 N and the sulfuric acid solution with a concentration of 28 N were added simultaneously until a preset amount of sodium silicate aqueous solution was added, wherein the sodium silicate aqueous solution had a preset amount of 42.5 L and a flow rate of 0.625 L/min; a pH value of a reaction solution was controlled to 9-10 and a temperature was controlled to 75 °C-80 °C during the reaction, and then the sulfuric acid solution was stopped adding for aging for 45 min; and
3) the sulfuric acid solution with a concentration of 28 N was added to the reaction tank until a pH value of a reaction solution was 4.5, then the sulfuric acid solution was stopped adding for aging for 15 min, and then drying and crushing were performed on an aged product to obtain the amorphous silica particle.

### Example 4

An amorphous silica particle, which was prepared by a preparation method as follows:
1) 8 L of water was added to a reaction tank and heated to 70 °C, and then stirring was started at a rotation speed of 1700 r/min; 4 L of sodium silicate aqueous solution with a concentration of 3.3 N was added, then the sodium silicate aqueous solution with a concentration of 3.3 N and a sulfuric acid solution with a concentration of 32 N were added simultaneously until a preset amount of sodium silicate aqueous solution was added, wherein the sodium silicate aqueous solution had a preset amount of 80 L and a flow rate of 1.33 L/min; a pH value of a reaction solution was controlled to 7-8 and a temperature was controlled to 70 °C-75 °C during the reaction, and then the sulfuric acid solution was stopped adding for aging for 60 min;
2) 1.6 L of sodium silicate aqueous solution with a concentration of 3.3 N was added to the reaction tank, then the sodium silicate aqueous solution with a concentration of 3.3 N and the sulfuric acid solution with a concentration of 32 N were added simultaneously until a preset amount of sodium silicate aqueous solution was added, wherein the sodium silicate aqueous solution had a preset amount of 40 L and a flow rate of 0.5 L/min; a pH value of a reaction solution was controlled to 9-10 and a temperature was controlled to 75 °C-80 °C during the reaction, and then the sulfuric acid solution was stopped adding for aging for 45 min; and
3) the sulfuric acid solution with a concentration of 32 N was added to the reaction tank until a pH value of a reaction solution was 4.5, then the sulfuric acid solution was stopped adding for aging for 15 min, and then drying and crushing were performed on an aged product to obtain the amorphous silica particle.

### Example 5

An amorphous silica particle, which was prepared by a preparation method as follows:
1) 10 L of water was added to a reaction tank and heated to 75 °C, and then stirring was started at a rotation speed of 1700 r/min; 5 L of sodium silicate aqueous solution with a concentration of 3.5 N was added, then the sodium silicate aqueous solution with a concentration of 3.5 N and a sulfuric acid solution with a concentration of 36 N were added simultaneously until a preset amount of sodium silicate aqueous solution was added, wherein the sodium silicate aqueous solution had a preset amount of 50 L and a flow rate of 1.66 L/min; a pH value of a reaction solution was controlled to 7-8 and a temperature was controlled to 70 °C-75 °C during the reaction, and then the sulfuric acid solution was stopped adding for aging for 60 min;
2) 1.5 L of sodium silicate aqueous solution with a concentration of 3.5 N was added to the reaction tank, then the sodium silicate aqueous solution with a concentration of 3.5 N and the sulfuric acid solution with a concentration of 36 N were added simultaneously until a preset amount of sodium silicate aqueous solution was added, wherein the sodium silicate aqueous solution had a preset amount of 30 L and a flow rate of 0.75 L/min; a pH value of the reaction solution was controlled to 9-10 and a temperature was controlled to 75°C-80°C during the reaction, and then the sulfuric acid solution was stopped adding for aging for 45 min; and
3) the sulfuric acid solution with a concentration of 36 N was added to the reaction tank until a pH value of a reaction solution was 4.5, then the sulfuric acid solution was stopped adding for aging for 15 min, and then drying and crushing were performed on an aged product to obtain the amorphous silica particle.

### Performance testing

1) The physical and chemical indexes of the amorphous silica particle prepared in Examples 1-5 are as shown in the following table.

**Table 1 Physical and chemical indexes of the amorphous silica particle prepared in Examples 1-5**

| **Physical and chemical indexes** | **Example 1** | **Example 2** | **Example 3** | **Example 4** | **Example 5** |
|---|---|---|---|---|---|
| **Oil factor (g/100mL)** | 86 | 73 | 62 | 50 | 45 |
| **Apparent density (g/mL)** | 0.39 | 0.48 | 0.53 | 0.57 | 0.58 |
| **BET specific surface** area **(m²/g)** | 70.0 | 60.8 | 55.7 | 41.1 | 38.4 |
| **CTAB specific surface area (m²/g)** | 60.00 | 54.11 | 51.83 | 39.04 | 37.63 |
| **CTAB specific surface area/BET specific surface area** | 0.86 | 0.89 | 0.93 | 0.95 | 0.98 |
| **Ra value (µm)** | 0.676 | 0.764 | 0.785 | 0.824 | 0.885 |
| **Radioactive Dentin Abrasivity (RDA) value** | 140 | 180 | 190 | 200 | 230 |
| **Pellicle Cleaning Ratio (PCR) value** | 87 | 98 | 109 | 115 | 120 |

| | | | | | |
|---|---|---|---|---|---|
| Notes: Oil factor: oil factor was tested with reference to the oil factor measuring method in "QB/T2346-2007 Silicon Dioxide for Toothpaste". BET specific surface area: the specific surface area, pore volume and pore diameter of the amorphous silica particle was test by the JW-BK112 static nitrogen adsorption apparatus. CTAB specific surface area: CTAB specific surface area was tested with reference to "GB/T 23656-2016 Rubber Compounding Ingredients - Determination of Surface Area for Silica, Precipitated, hydrated - CTAB Method". Ra value: Ra value was tested with reference to "GB/T 35832-2018 Testing Method of the Abrasivity Value of Toothpastes". RDA value: the abrasiveness of tooth powder in dentin was measured with the Hefferren grinding test, recommended by ADA and ISO (the sample was sent to the Indiana University in the USA for testing). PCR value (Pellicle Cleaning Ratio): PCR value was tested with reference to "T/COCIA7-2020 Laboratory Evaluation Method of Toothpaste to Remove Exogenous Stains". | | | | | |

It can be seen from Table 1 that the CTAB specific surface area/BET specific surface area of the amorphous silica particle in Examples 1-5 are close to 1, both the micropore and macropore are less in quantity, and friction and cleaning properties are excellent (The closer the CTAB specific surface area/BET specific surface area is to 1, the better the performance).

2) A toothpaste (toothpaste formula: 57wt% sorbitol, 15.4wt% distilled water, 0.8wt% carboxymethylcellulose, 8wt% thickening silica, 15wt% amorphous silica particle, 0.2wt% sodium benzoate, 0.2wt% sodium saccharin, 2.2wt% sodium lauryl sulfate, 1wt% essence and 0.2wt% titanium dioxide) was prepared with each of the amorphous silica particle prepared in Examples 1-5 as an abrasive, and then a performance test was performed on the toothpaste, with the test results as shown in the following table.

**Table 2 Performance test results of toothpastes prepared with the amorphous silica particles prepared in Examples 1-5**

| **Performance index** | **Ra value (µm)** | **Transparency** | **Comprehensive evaluation** |
|---|---|---|---|
| **Example 1** | 0.302 | 9-10 | Medium-to-high friction and cleaning properties, and a higher transparency |
| **Example 2** | 0.781 | 9-10 | A higher RDA value and a higher PCR value, and excellent friction and cleaning properties |
| **Example 3** | 0.831 | 10-11 | A higher RDA value and a higher PCR value, and excellent friction and cleaning properties |
| **Example 4** | 0.858 | 10-11 | A higher RDA value and a higher PCR value, excellent friction and cleaning properties, and an obvious grain taste in the toothpaste |
| **Example 5** | 0.893 | 11-12 | A higher RDA value and a higher PCR value, excellent friction and cleaning properties, and a high transparency |

| | | | |
|---|---|---|---|
| Notes: Transparency: transparency was tested with reference to the light transmittance measuring method in "QB/T2346-2007 Silicon Dioxide for Toothpaste". | | | |

The transparent toothpaste is transparent because a liquid phase and a solid phase of the paste have a similar refractive index, and if their refractive indexes reach the same, the transparency or light transmittance may reach 100%. The main ingredients of the liquid phase of the toothpaste are sorbitol, water, polyethylene glycol (PEG) and glycerol, and the liquid phase and the solid phase may have the same refractive index by regulating a ratio of the water to a wetting agent. When the liquid phase and the solid phase have the same refractive index, the silica particle is dispersed in a hydrophilic liquid phase, a diffuse reflection will not generate, thereby forming the paste with a better transparency and obtaining the effect of a transparent appearance. 70wt% sorbitol has a refractive index of 1.45-1.46, the water has a refractive index of 1.333, the PEG has a refractive index of 1.46, and the glycerol has a refractive index of 1.47. Generally speaking, the refractive index of the silica in the range of 1.435-1.455 is better.

It can be seen from Table 2 that, serving as an abrasive, the amorphous silica particle provided by the present disclosure may be used in the toothpaste with higher friction and cleaning properties and higher transparency, without causing excessive wear of dentin and enamel, so the amorphous silica particle is the preferred abrasive for making the high-cleaning, whitening and transparent toothpaste.

The above examples are preferred examples of the present disclosure, but the implementations of the present disclosure are not limited to the above examples. Any other changes, modifications, substitutions, combinations and simplifications made without departing from the gist and principles of the present disclosure should be equivalent substitutions, and are all included in the protection scope of the present disclosure.

## Claims

1. An amorphous silica particle, wherein the amorphous silica particle meets 0.85≤a Cetyl Trimethyl Ammonium Bromide (CTAB) specific surface area/a Brunauer-Emmett-Teller (BET) specific surface area≤1.

2. The amorphous silica particle according to claim 1, wherein the CTAB specific surface area of the amorphous silica particle is ≤60 m²/g.

3. The amorphous silica particles according to claim 2, wherein the BET specific surface area of the amorphous silica particle is ≤70 m²/g.

4. The amorphous silica particle according to any one of claims 1 to 3, wherein an apparent density of the amorphous silica particle is ≥0.1 g/mL.

5. The amorphous silica particle according to any one of claims 1 to 3, wherein an oil factor of the amorphous silica particle is ≤300 g/100 g.

6. A preparation method for the amorphous silica particle according to any one of claims 1 to 5, comprising the following steps:
1) adding a certain amount of water to a reaction tank, heating to 50 °C-90 °C and then starting stirring; adding a certain amount of sodium silicate aqueous solution, and then adding the sodium silicate aqueous solution and a sulfuric acid solution simultaneously at a maintained flow rate until a preset amount of sodium silicate aqueous solution is added, controlling a pH value of a reaction solution to 7-8 and a temperature to 50 °C-75 °C during the reaction, and then stopping adding the sulfuric acid solution for aging;
2) adding a certain amount of sodium silicate aqueous solution to the reaction tank, and then adding the sodium silicate aqueous solution and the sulfuric acid solution at a maintained flow rate until a preset amount of sodium silicates aqueous solution is added, controlling a pH value of a reaction solution to 9-10 and a temperature to 75 °C-90 °C during the reaction, and then stopping adding the sulfuric acid solution for aging; and
3) adding the sulfuric acid solution to the reaction tank until a pH value of a reaction solution is 4-5, and then stopping adding the sulfuric acid solution for aging; drying and then crushing an aged product to obtain the amorphous silica particle.

7. The preparation method according to claim 6, wherein the certain amount of water, the certain amount of sodium silicate aqueous solution and the preset amount of sodium silicate aqueous solution in step 1) have a volume ratio of 1:0.4-0.6:5-10.

8. The preparation method according to claim 6, wherein the sodium silicate aqueous solution in step 1) has a concentration of 2.7 N-3.5 N, the sulfuric acid solution in step 1) has a concentration of 18 N-36 N, and the aging in step 1) lasts for 15 min-60 min.

9. The preparation method according to claim 6, wherein the certain amount of sodium silicate aqueous solution and the preset amount of sodium silicate aqueous solution in step 2) have a volume ratio of 1: 15-25.

10. The preparation method according to claim 6, wherein the preset amount of sodium silicate aqueous solution in step 1) and the preset amount of sodium silicate aqueous solution in step 2) have a volume ratio of 1:0.5-4.

11. The preparation method according to claim 6, wherein the sodium silicate aqueous solution in step 2) has a concentration of 2.7 N-3.5 N, the sulfuric acid solution in step 2) has a concentration of 18 N-36 N, and the aging in step 2) lasts for 15 min-60 min; the sulfuric acid solution in step 3) has a concentration of 18 N-36 N, and the aging in step 3) lasts for 15 min-60 min.

12. An oral cleaning composition, comprising the amorphous silica particle according to any one of claims 1 to 5.

13. The oral cleaning composition according to claim 12, wherein the oral cleaning composition further comprises an orally acceptable carrier.

14. The oral cleaning composition according to claim 12 or 13, wherein the amorphous silica particle has a weight percentage of 10%-30%.

15. The oral cleaning composition according to claim 12 or 13, wherein the oral cleaning composition is a toothpaste.
